# EUROPEAN PATENT APPLICATION

(11) **EP 2 380 618 A1**
(43) Date of publication of application: **26.10.2011**
(21) Application number: 10161013.7
(22) Date of filing: 26.04.2010
(51) Int. Cl.: A61M 11/00, A61M 15/00, A61M 15/08

(54) **Operating method for an aerosol delivery device and aerosol delivery device**

(71) Applicant: PARI Pharma GmbH, 82319 Starnberg (DE)
(72) Inventor: Krüner, Axel, 80802 München (DE); Klopfer, Elisabeth, 83109 Grosskarolinenfeld (DE); Schuschnig, Uwe, 81371 München (DE); Seifert, Rene, 82418 Murnau (DE)
(74) Representative: HOFFMANN EITLE

(57) **Abstract**

The invention relates to a method for operating an aerosol delivery device (10), comprising the steps of generating a predetermined amount of an aerosol in the device (10) and inducing a transport flow in the device (10) for transporting at least a portion of the predetermined amount of the aerosol outside the device (10). During the aerosol generating step, the transport flow is first induced, then stopped after a predetermined first period of time and then induced again after a predetermined second period of time. Further, the invention relates to an aerosol delivery device (10) comprising an aerosol generator (3) for generating an aerosol in the device (10), a gas conveying element (1) for inducing a transport flow in the device (10) for transporting at least a portion of the generated aerosol outside the device (10), and a control configured, during the aerosol generating step, to first activate the gas conveying element (1), then deactivate the gas conveying element (1) after a predetermined first period of time and then activate the gas conveying element (1) again after a predetermined second period of time, so that the transport flow is first induced, then stopped after the first period of time and then induced again after the second period of time.

## Description

### Field of the Invention

The invention relates to a method for operating an aerosol delivery device (nebuliser) and an aerosol delivery device implementing this method.

### Background Art

Diseases and conditions affecting either paranasal sinuses or both the nasal cavity and the paranasal sinuses, in particular acute and chronic forms of rhinosinusitis, are increasing in incidence and prevalence in many countries and regions of the world, including Europe and the United States. These conditions may be associated with significant symptoms and have a negative impact on quality of life and daily functioning.

The method most commonly used to deliver medications to the nasal cavity is a squeeze bottle or a metering spray pump nebulising volumes of 50 to 140 µl per actuation. However, studies investigating the *in vivo* deposition pattern of droplets administered by a spray pump indicate that local distribution is primarily in the anterior portion of the nasal cavity leaving large portions of the nasal cavity unexposed to drug (see Suman et al., "Comparison of nasal deposition and clearance of aerosol generated by a nebuliser and an aqueous spray pump", Pharmaceutical Research, Vol. 16, No. 10, 1999). Furthermore, drugs applied by nasal pump sprays are cleared very fast from the nose, an average clearance time of between 10 and 20 minutes being accepted as normal (see C. Marriott, "Once-a-Day Nasal Delivery of Steroids: Can the Nose Be Tricked?" RDD Europe 2007, proceedings p.179-185). The fast clearance rate of the nose and the difficulties to overcome these disadvantages by an increase of the solution viscosity have also been described by Pennington et al. ("The influence of solution viscosity on nasal spray deposition and clearance", Intern. Journal of Pharmaceutics, 43, p. 221-224, 1988). However, those attempts were only successful to improve retention of drugs in the nose prolonging the residence time, the time to clear 50% of dose, up to 2.2 hours. Consequently, the effective treatment of the nasal and paranasal mucosa via a method to increase residence time remains challenging. While the mucosa of the nasal cavity is a feasible target for locally administered drugs formulated as nasal sprays, the sinuses and the osteomeatal complex are not easily accessed by liquid formulations. In the case of relatively coarse aerosols, such as conventional nasal sprays, the deposition on the sinus mucosa is negligible, and even finer aerosols, such as those generated by nebulisers, exhibit a very low degree of sinus deposition.

The primary reason for the lack of access of an inhaled aerosol to the sinuses is anatomical: in contrast to the nasal cavity, the sinuses are not actively ventilated. The latter are connected to the nasal passage via small orifices called ostia, whose diameter is typically in the region of about 0.5 to 3.0 mm for a healthy person and up to about 10 mm for a patient after sinus surgery (functional endoscopic sinus surgery). When air is inhaled through the nose and passes through the nasal passage into the trachea, there is only very little convective flow into the ostia.

To address the need for devices and methods which are more effective in delivering an aerosol to the osteomeatal complex and paranasal sinuses, it was suggested in WO 2005/023335 that certain particle size and vorticity characteristics must be achieved in order that a majority of an aerosolised drug formulation reaches the deep nasal cavities and the sinuses. Furthermore, WO 2004/020029 discloses an aerosol generator comprising a nebuliser and a compressor which delivers a pulsating stream of air to the nebuliser. In use of this aerosol generator, the main aerosol flow supplied to a patient's nostril is superimposed by pressure fluctuations in order to improve the aerosol deposition efficiency in the paranasal sinuses. This document further describes that the aerosol emitted from the nebuliser should be introduced through one nostril via an appropriate nosepiece with closed soft palate, and that the contralateral nostril should be closed by an appropriate flow resistance device.

A substantial further improvement was achieved through the teaching of EP 1 820 493 A2 according to which the sinunasal deposition of a pulsating aerosol can be significantly increased if it is ensured that the pressure fluctuation maintains a certain amplitude, such as at least about 5 mbar pressure difference. The used frequencies are around 10 Hz to 90 Hz.

Nevertheless, it is still only a fraction of any aerosol which can be delivered to the sinunasal target area by the methods known today. Furthermore, there exists a problem in known methods that the pressure oscillations or pulsations superimposed on the main aerosol flow lead to an increased aerosol impaction on the walls of the aerosol generator (aerosol delivery device) and/or the nostril entry, resulting in a reduced aerosol output and consequently a less efficient therapeutic treatment. In addition, there remains a need for a simplified aerosol delivery method and device, eliminating the requirement of an additional flow resistance device and the closure of the soft palate.

### Summary of the Invention

One object of the invention is to provide a simple method for operating an aerosol delivery device that may increase the fraction of any generated aerosol delivered to the sinunasal target area, consequently offering a more efficient therapeutic treatment. Further, the invention aims to provide an aerosol delivery device implementing this method. These goals are achieved by a method with the technical features of claim 1 and a device with the technical features of claim 9. preferred embodiments of the invention follow from the dependent claims.

The invention provides a method for operating an aerosol delivery device, comprising the steps of generating a predetermined amount of an aerosol in the device, and inducing a transport flow in the device for transporting at least a portion of the predetermined amount of the aerosol outside the device. During the aerosol generating step, the transport flow is first induced, then stopped after a predetermined first period of time and then induced at least once again after a predetermined second period of time. Herein, a transport flow may be induced in the device only during the aerosol generating step or, alternatively, during and after the aerosol generating step. The method may include one or a plurality of cycles of inducing, stopping and inducing again the transport flow. The method of the invention is not to be used with a respirator or a resuscitation apparatus.

By performing at least one such cycle, the amount of aerosol deposited in a desired location outside the aerosol delivery device, such as the nasal cavity, the mucosa in the nose or, in particular, the paranasal sinuses, can be significantly increased as compared to a delivery (generation, inhalation) device operation where the transport flow is held constant. Such an intermittent transport flow exploits the inertia of the generated aerosol and is particularly efficient for improving aerosol deposition in the paranasal sinuses, especially for the case of enlarged ostia with diameters of the order of 10 mm, e.g., for a patient after functional endoscopic sinus surgery (post FESS). In this way, a more efficient therapeutic treatment can be provided.

Further, since no additional pulsation (vibration) has to be superimposed onto the transport flow during aerosol generation, in order to achieve efficient aerosol deposition, the method of the invention is particularly simple and allows for the use of an aerosol delivery device with a simple, common structure, requiring only a single motor.

The absence of such additional pulsations during the aerosol generating step further provides the advantage that the impaction of aerosols on the walls of the aerosol delivery device can be largely prevented, resulting in a reduced loss of aerosols in the device and consequently an increased aerosol output at the desired location. Moreover, the nose is a very efficient particle filter with narrow cross sectional areas, leading to a high fraction of such pulsated aerosol being deposited in the anterior and central nasal regions (see W. Möller et al, "Human Nasal DTPA Clearance and Systemic Absorption after Pulsating Aerosol Delivery Using the Pari Sinus", RDD 2008, p. 553-556). Hence, the aerosol losses in the device and areas of the nose other than the paranasal sinuses can be reduced, thus further improving therapeutic efficiency.

Preferably, the transport flow is an air flow. In this case, the transport flow can be provided in particularly simple manner with a gas conveying element (or transport flow producer, like a pump), using, for example, ambient air, so that no separate gas reservoir is required.

The flow rate of the transport flow is preferably up to 10 L/min, more preferably up to 5 L/min and even more preferably between 0.5 and 3 L/min. The pressure drops for such low flow rates are relatively small. For example the pressure value is below 5 mbar, when measured in the nasal cast. The transport flow rate can be measured by using a conventional flow sensor with short response times (e.g., 1-3 ms), such as the Flow Sensor FBAL001DU from Sensortechnics.

In one embodiment, the transport flow is periodically induced and stopped during the aerosol generating step, preferably at least three times. The frequency of periodically inducing and stopping the transport flow is preferably less than 10 Hz, more preferably between 1 and 10 Hz, even more preferably less than 3 Hz and yet even more preferably between 1 and 3 Hz, so as to achieve particularly efficient aerosol deposition. However, the time intervals between different inductions of the transport flow may also vary, depending, for example, on the type of aerosol used. By increasing the number of cycles of inducing, stopping and inducing again the transport flow, the efficiency of the aerosol deposition can be further improved.

Preferably, the first period of time and/or the second period of time is in the range of 50 - 300 ms. Herein, the first and second periods of time may be identical or different from each other. Preferably, the first period of time is larger than the second period of time.

Preferably, the total volume of transported gas/aerosol bolus during one application cycle (period step) is less than 50 ml more preferably between 5 ml and 20 ml. Herein, the term "total volume of gas/aerosol bolus" refers to the entire amount of gas volume, which contains aerosol as a mixture, that is transported outside the device when performing the method of the invention.

The method of the invention may further comprise a step of pulsating (vibrating) the aerosol after the aerosol generating step. As used herein, the term "pulsation (vibration, flow and pressure oscillation) of an aerosol" is understood as a periodic change of flow rate (or aerosol flow) that occurs at a predetermined frequency. An example for a typical pulsating flow V_{pulsating} (t) (or pulsation flow or pulsatile flow rate) is a sinusoidal function like: V_{pulsating}(t) = V_{constant} + V_{variablerMax}* sin (2π*frequency*time) . The PARI SINUS device for example provides a constant sinusoidal airflow with a constant pulsation flow V_{constant}= 7 L/min, a maximal pulsation flow V_{variablerMax}= 11 L/min, and a frequency=44 Hz. Preferably, the pulsation is regular, i.e., the time interval between flow peaks is approximately constant. The amplitude of the pulsations may also be substantially constant. By pulsating the aerosol at a given frequency, aerosol diffusion can be significantly enhanced, enabling improved access to locations that are difficult to reach with a constant aerosol flow, such as the paranasal sinuses. Additionally, flow fluctuations induce also pressure fluctuations in the nose as the nasal passage represents a flow resistor. These pressure differences between nasal and sinus cavity effectuate an airflow and with it, ventilation of the sinuses. The principle of applying a pulsating aerosol for enhanced sinus deposition has recently been found and is described, for example, in WO 2004/020029.

Such aerosol pulsations are particularly effective for enhancing aerosol deposition into the paranasal sinuses through ostia with a small diameter of about 0.5 to 3.0 mm, which is a typical range for patients before sinus surgery. On the other hand, as has been discussed above, the provision of an intermittent transport flow during aerosol generation is particularly effective for improving aerosol deposition in the paranasal sinuses for the case of enlarged ostia with diameters of the order of 10 mm, e.g., for patients after functional endoscopic sinus surgery (post FESS). By combining these two approaches, i.e., intermittent transport flow during and superimposed pulsation after aerosol generation, efficient aerosol deposition can be ensured over the whole range of possible ostia diameter, thus further improving the efficiency of therapeutic treatment.

Since the aerosol is only pulsated after the aerosol generating step, aerosol losses in the aerosol delivery device and areas of the nose other than the sinuses can be reduced. In particular, the aerosol deposition in the anterior part of the nose, the nasal valve and the nasal vessel can be significantly reduced. Preferably, the transported aerosol is pulsated when it has reached a desired location outside the aerosol delivery device, such as the paranasal sinuses. More preferably, the transported aerosol is pulsated only when it has reached this location, so that such aerosol losses can be further decreased. Moreover, by stopping the aerosol generation before a pulsation is induced, a possible effect of the pulsation on the aerosol generation process can be avoided.

In other embodiments, different combinations of the intermittent mode, i.e., the provision of an intermittent transport flow during aerosol generation, and the pulsation mode, i.e., the superposition of pulsations after aerosol generation, can be used. For example, the aerosol delivery device may be operated twice in the intermittent mode, followed by one operation or two operations in the pulsation mode, or the aerosol delivery device may be operated once in the intermittent mode, followed by one operation or two operations in the pulsation mode. Further, e.g., the following combinations of intermittent mode (I) and pulsation mode (V) may be employed: once (I) followed by three times (V); once (I) followed by four times (V); once (I) followed by five times (V); three times (I) followed by once (V); four times (I) followed by once (V) or five times (I) followed by once (V).

Further, the transport flow may be stopped during the step of pulsating the aerosol, leading to a further reduction of such aerosol losses.

Preferably, the duration of the step of pulsating the aerosol is in the range of 0.1 - 15.0 s, more preferably in the range of 0.5 - 1.0 s.

The pulsation of the aerosol may have a frequency in the range of 1 - 200 Hz. According to some further embodiments, the aerosol may also be pulsated at a frequency of at least about 20 Hz, at least about 40 Hz, at least about 60 Hz or at least about 100 Hz, respectively.

In one embodiment, the pulsation of the aerosol has a flow amplitude in the range of 0 to 20 L/min (preferably of the order of 20 L/min) by passing the output channel of the aerosol delivery device. It has been found that, depending on the individual anatomy of a human person, the flow amplitude results in different flow velocity (and pressure) values on the way to the desired location. The anatomy of the entrance area to the paranasal sinus has a great influence on the resulting air flow velocity, which transports the aerosol in the desired deposition target areas in the paranasal sinuses. This may be influenced by the flow channel size through the nose; the different ostia diameters to the paranasal sinuses and the size of the paranasal sinus volumes. For example, small ostia diameters will reduce the flow through the ostia to the paranasal sinuses. For example, if a flow of 10 L/min is chosen, the flow of the pulsation (vibration, fluctuation) through the ostia can periodically vary between -20 L/min and +20L/min. Alternatively, the amplitude of the aerosol pulsation may be maintained at a level of at least about 5 L/min, or at least about 10 L/min, or at least about 15 L/min by passing the output channel of the aerosol delivery device. Preferably, the pulsation of the aerosol, in use, has a maximal pulsation flow (V_{variablerMax}) greater than 8 L/min, more preferably greater than 12 L/min and even more preferably greater than 14 L/min.

The pulsation flow rate (flow amplitude) can be measured by using a conventional flow sensor with short response times (e.g., 1-3 ms) which is capable of measuring both positive and negative flows (i.e., suitable for measuring pulsation flows), such as the Flow Sensor FBAL001DB from Sensortechnics.

Preferably, the generation, transportation and pulsation of the aerosol are independent of a further flow (gas flow), such as an inhalation, exhalation or breathing manoeuvre (process) of a patient.

In one embodiment, the aerosol is a pharmaceutical aerosol for the delivery of an active compound. An active compound is a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active pharmaceutical ingredient, drug substance, drug, and the like.

The active compound comprised in the aerosol used for the method of the invention may be a drug substance which is useful for the prevention, management, or treatment of any disease, symptom, or condition affecting the nose, the sinuses and/or the osteomeatal complex, nasal or sinunasal conditions caused by lower respiratory tract diseases, and nasal or sinunasal conditions caused by ear diseases. The method of the invention achieves a highly efficient deposition of the active compound in the nasal cavities, the paranasal sinuses, the ear, and/or the respiratory system. Thus, it may be advantageously used for the prevention, management, or treatment of the above diseases, symptoms or conditions. In addition, the present method may also be used to deliver active compounds to the systemic circulation or to the brain for prevention, management, or treatment of any systemic or brain disease, symptom, or condition. Among the active compounds which may be useful for serving one of these purposes are, for example, substances selected from the group consisting of anti-inflammatory compounds, anti-infective agents, antiseptics, prostaglandins, endothelin receptor agonists, phosphodiesterase inhibitors, beta-2-sympathicomimetica, decongestants, vasoconstrictors, anticholinergics, immunomodulators, mucolytics, anti-allergic drugs, antihistaminica, mast-cell stabilizing agents, tumor growth inhibitory agents, wound healing agents, local anaesthetics, antioxidants, oligonucleotides, peptides, proteins, vaccines, vitamins, plant extracts, phosharimidon, vasoactive intestinal peptide, serotonin receptor antagonists, and heparins.

Further, it may be advantageous to provide a low frequency pulsation to assist mucus clearance and/or ciliary movement (for example the Nasal mucocilliary Clearance - NMCC). This low frequency pulsation may either be generated during transport flow, inbetween two consecutive transport flows (pause) and/or during the step of pulsating the aerosol mentioned above as an additional pulsation (for example as a heterodyne frequency). In this context, the selected frequency is preferably less than 60 Hz, more preferably between 5 Hz and 40 Hz and most preferred between 10 to 20 Hz. Particular preferred, in this regard may be a frequency of 16 Hz. Such a frequency has already been proven advantageous with respect to Mucus clearance in applications treating the lungs as described in EP 0 565 489 B1, US 6,984,214 B2 or US 6,702,769 B1. Also WO 03/059237 A1 may be referred to in this regard. Finally, it is to be noted that this particular feature may even be implemented in an aerosol delivery device without the intermittent transport flow, that is an aerosol delivery device providing a constant transport flow with or without an additional pulsation. The aerosol delivery device may comprise a pulsator for pulsating the aerosol and a control which is configured to activate the pulsator for performing a step of low-frequency pulsation with a frequency in the range of less than 60 Hz, preferably between 5 to 40 Hz, most preferred between 10 and 20 Hz.

The invention further provides an aerosol delivery device comprising an aerosol generator for generating an aerosol in the device, a gas conveying element (or transport flow producer, such as a pump) for inducing a transport flow in the device for transporting at least a portion of the generated aerosol outside the device, and a control configured, during the aerosol generating step, to first activate the gas conveying element, then deactivate the gas conveying element after a predetermined first period of time and then activate the gas conveying element at least once again after a predetermined second period of time, so that the transport flow is first induced, then stopped after the first period of time and then induced at least once again after the second period of time. Such an aerosol delivery device can be efficiently used to perform the method of the invention. For example the gas conveying element (or transport flow producer) will be designed as a pump, compressor, compressed air supply, turbine and/or ventilator (with included work modes like BiPAP, CPAP, ASB, PAV and so on), which works with or without Venturi principle, gas valve(s), gas controller, regulator, actuator, interrupter, switch three way selector valve. Further, the device has a simple configuration and can be operated using only a single motor. The aerosol delivery device of the invention is not a respirator or a resuscitation apparatus.

Preferably, the control is configured to, preferably at least three times, periodically activate and deactivate the gas conveying element during the aerosol generating step, so that the transport flow is periodically induced and stopped accordingly. Here, the control may be configured so that the frequency of periodically activating and deactivating the gas conveying element is less than 10 Hz, preferably between 1 and 10 Hz, more preferably less than 3 Hz and even more preferably between 1 and 3 Hz.

Preferably, the control is configured so that the first period of time and/or the second period of time is in the range of 50 - 300 ms. Herein, the control may be configured so that the first and second periods of time are identical or different from each other. Preferably, the control is configured so that the first period of time is larger than the second period of time.

Preferably, the control is configured so that the total volume of transported gas/aerosol bolus during one application cycle is less than 50 ml, more preferably between 2 ml and 20 ml.

The control may be configured so that the flow rate of the transport flow is up to 10 L/min, more preferably up to 5 L/min and even more preferably between 0.5 and 3 L/min.

The aerosol delivery device of the invention may further comprise a pulsator (vibrator) for pulsating (vibrating) the aerosol, wherein the control is configured to activate the pulsator after deactivating the aerosol generator. In this way, the aerosol delivery device can be operated in a mode which is a combination of intermittent mode and pulsation mode, as discussed above. In particular, the control may be configured to operate the device in any conceivable mode which combines intermittent and pulsation modes, including the example modes described above. Further, the control may be configured to activate the pulsator for performing a step of low-frequency pulsation with a frequency in the range of less than 60 Hz, preferably between 5 to 40 Hz, most preferred between 10 and 20 Hz.

The control may further be configured to deactivate the gas conveying element (or transport flow producer) during the pulsation of the aerosol.

Preferably, the aerosol generator is a vibrating membrane nebuliser. In this case, no transport flow is required for the generation of an aerosol so that aerosol generation and transport are entirely independent from each other. Therefore, the transport flow during aerosol generation can be precisely controlled and efficiently stopped. In this way, the aerosol deposition efficiency can be further improved.

The aerosol delivery device of the invention may further comprise a switch unit for switching the control between an operating mode which includes activating the pulsator after deactivating the aerosol generator, e.g., one of the combined modes described above, and an operating mode in which the pulsator is not activated, i.e., a pure intermittent mode. In this way, the device is widely usable for a variety of applications, the switch unit allowing for a quick, simple and convenient change between the different modes. Since only a single device is required for different operation modes, such a configuration is further very cost efficient. The switch unit may further be configured to enable switching to a third mode, e.g., a pure pulsation mode. The switch unit may, for example, be a mechanical or electronic switch or a memory device, such as a FlashCard, a SmartCard, a USB-stick, a key card etc. Furthermore, switching could also be performed remotely through a data connection, e.g., via Bluetooth, Infrared, a mobile phone chip, a memory device in the processing unit etc.

The aerosol delivery device of the invention may further comprise a sensor element for sensing periods of exhalation of a patient. This sensor element may be further configured to allow aerosol transport outside the device only during such a period of exhalation, e.g., by suitably triggering the gas conveying element (like a pump), controlling a suitable valve etc. By allowing an aerosol transport flow only during a period of exhalation, the ventilation of the paranasal sinuses is improved, resulting in an even more efficient deposition of the transported aerosol in the paranasal sinuses. If such a sensor element for automatically triggering the aerosol transport flow is used, the aerosol deposition process can be carried out in a well-defined and controlled manner without the need for any coordination efforts from the patient. The sensor element may comprise a separate mouthpiece or nosepiece with a differential pressure sensor. Instead of a differential pressure sensor, flow sensor, pneumotachograph, flow controller, a pipe, a rotatable wheel, moveable flag, bi-metal sensor, piezoelectric element or an optical sensor may alternatively be employed for sensing the exhalation air flow.

The aerosol delivery device of the invention can be advantageously used to perform the method according to the invention.

### Brief Description of the Drawings

Hereinafter, non-limiting examples are explained with reference to the drawings, in which:
Figure 1 shows a schematic view of an aerosol delivery device according to an embodiment of the present invention;
Figure 2 shows a schematic view of an aerosol delivery device according to another embodiment of the present invention;
Figure 3 shows a schematic view of an aerosol delivery device according to yet another embodiment of the present invention;
Figure 4 shows a longitudinally cut cross-sectional view of the aerosol delivery device schematically shown in Fig. 1;
Figure 5 shows a flow diagram illustrating a possible operation of the aerosol delivery devices shown in Figs. 1 to 4 in the intermittent mode;
Figure 6 shows a flow diagram illustrating another possible operation of the aerosol delivery devices shown in Figs. 1 to 4 in a combined mode;
Figure 7 shows a diagram presenting experimental data on aerosol deposition efficiency for an aerosol delivery device operation as shown in Fig. 5 and Fig. 6; and
Figure 8 shows a perspective view of a set-up for measuring the pulsation flow rates of the aerosol delivery devices shown in Figs. 1 to 4.

### Detailed Description of Currently Preferred Embodiments

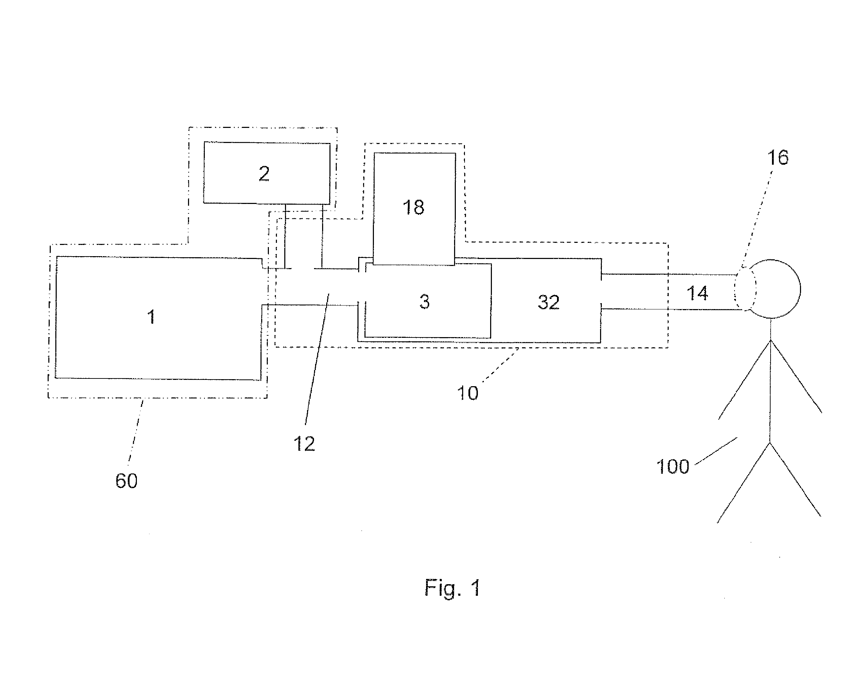

Figures 1 to 4 show schematic views of aerosol delivery devices 10 according to currently preferred embodiments of the present invention.

The aerosol delivery device 10 contains an aerosol generator 3, which may be an inhaler, atomiser or nebuliser, especially a nebuliser operating with a vibrating membrane or pores of a defined size.

As can be seen from Figs. 1 to 4, the aerosol delivery device 10 according to the currently preferred embodiments comprises a connector 12 for connection with a gas compressor 1 as a source of compressed air and an adaptation element 14 that is equipped with a nosepiece 16 or an optional mouthpiece 50 for adaptation to (communication with) a patient's 100 respiratory system, nasal cavity etc. A fluid container 18 for receiving a fluid to be nebulised is disposed between connector 12 and adaptation element 14. The fluid container 18 is preferably integrally formed with the body of the aerosol delivery device 10 but, in further embodiments, may be configured such that it is partly or fully detachable from the body. The body of the aerosol delivery device 10 is preferably made of plastic and preferably manufactured by an injection moulding process. The container 18 may be designed so that it does not directly receive the fluid but rather has an element, such as a spike, arranged on its inside that opens a fluid containing vessel, (e.g., a vial, blister, ampoule, container, canister, reservoir, cartridge, pot, tank, pen, storage, syringe) inserted therein.

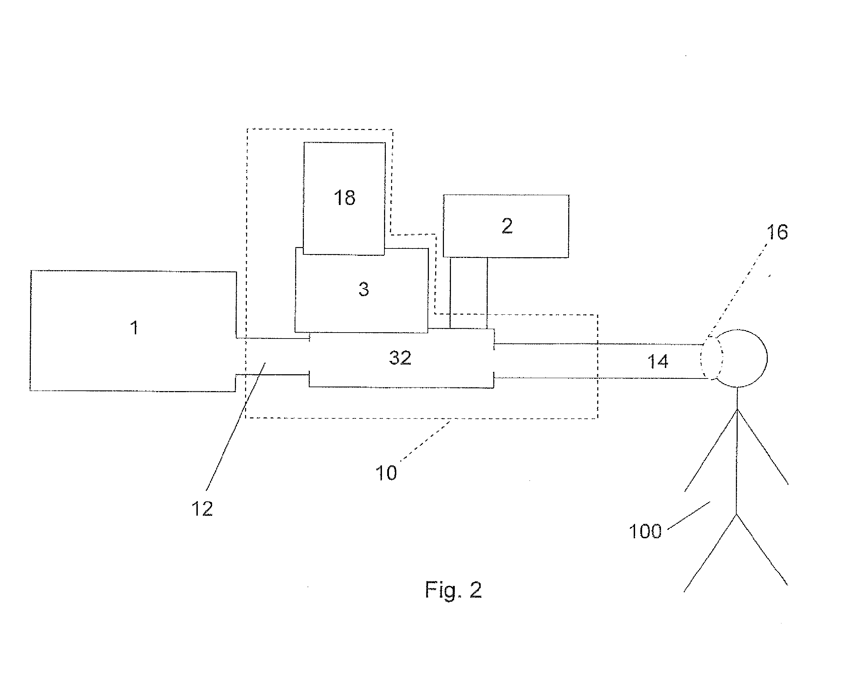

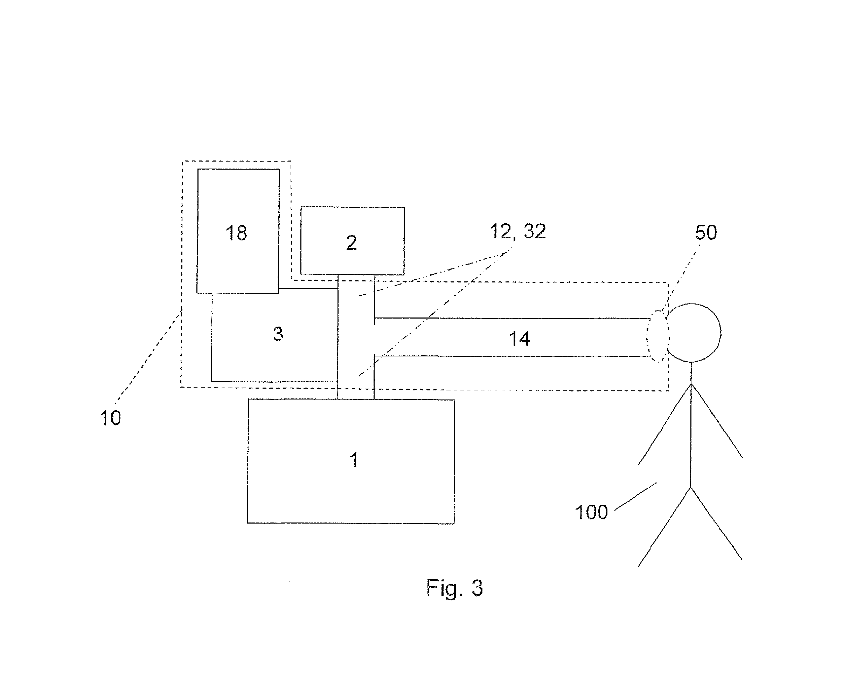

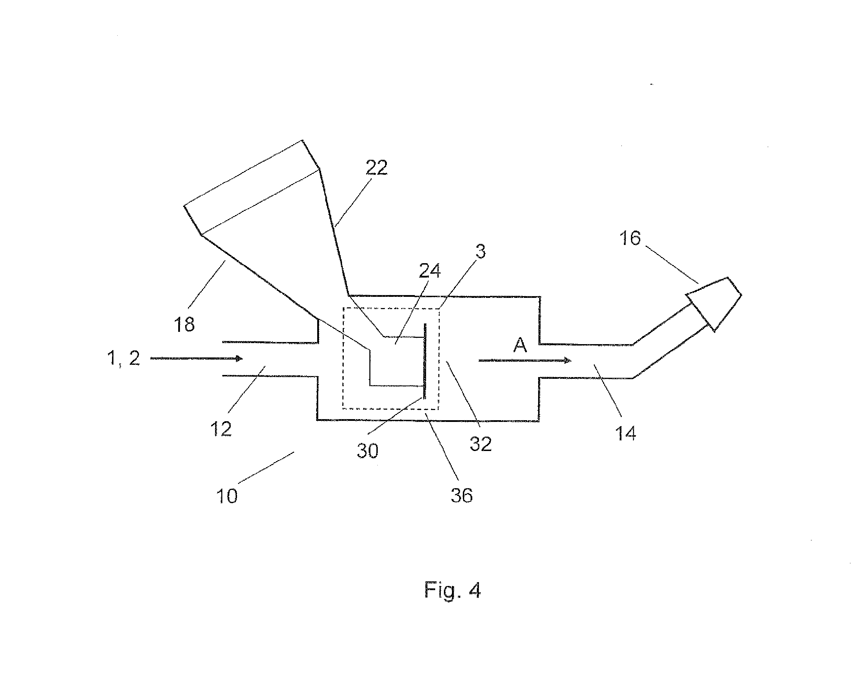

In the embodiments shown in Figs. 1 to 4, a gas compressor 1 is used as the gas conveying element and a sinus wave generator that is also connected to the connector 12 in the embodiments shown in Figs. 1, 3 and 4 is optionally used as the pulsator 2 in a combined mode, as will be further explained in the following. In the embodiment of Fig. 2, the sinus wave generator is connected to a nebuliser chamber 32 that is in fluid communication with the connector 12 and the adaptation element 14. In the embodiment of Fig. 3, the connector 12 and the nebuliser chamber 32 are integrally formed. The pulsator 2 and the gas compressor 1 of the embodiment shown in Figs. 1 and 4 together form a gas supply unit (air supply unit) 60.

In general, any aerosolisable fluid may be received in the fluid container 18 and used for the generation of an aerosol, depending on the condition, diagnoses to be measured or disease to be treated or managed in the aerosol delivery device. The fluid composition may comprise one or more active compounds, for example, substances selected from the group consisting of anti-inflammatory compounds, anti-infective agents, antiseptics, prostaglandins, endothelin receptor agonists, phosphodiesterase inhibitors, beta-2-sympathicomimetica, decongestants, vasoconstrictors, vasodilators, anticholinergics, immunomodulators, mucolytics, anti-allergic drugs, antihistaminica, leukotriene antagonists, mast-cell stabilizing agents, tumor growth inhibitory agents, wound healing agents, local anaesthetics, antioxidants, oligonucleotides, peptides, proteins, vaccines, vitamins, plant extracts, drugs obtained from fungi, antidepressant drugs, angiotensin converting enzyme inhibitors, platelet activating factor inhibitors, potassium channel openers, tachykinin and kinin antagonists, statins, calcium antagonists, drugs targeting cell signaling, phosharimidon, vasoactive intestinal peptide, serotonin receptor antagonists, and heparins.

Examples of potentially useful anti-inflammatory compounds are glucocorticoids such as alclomethasone, amcinonide, betamethasone, beclomethasone, budesonide, ciclesonide, clobetasole, clobetasone, clocortolone, desonide, dexamethasone, desoxymethasone, diflorasone, diflucortolone, fluoconolone acetonide, flucinonide, fludroxycortide, flumetasone, flunisolide, fluticasone, fluocinonide, fluocortinbutyl, fluocortolone, fluprednidene, halcinonide, halometasone, hydrocortisone, hydroxycortisone, icomethasone, methylprednisolone, mometasone, prednicarbate, prednisolone, prednisone, rofleponide, and triamcinolone acetonide; non-steroidal glucocorticoid receptor activators, such as dehydroepiandrosterone and derivatives such as dehydroepiandrosterone sulfat (DHEAS); non-steroidal anti-inflammatory agents, such as aceclofenac, acemetacin, bromfenac, diclofenac, etodolac, ibuprofen, indometacin, nabumetone, sulindac, tolmetin, carprofen, fenbufen, fenoprofen, flurbiprofen, ketoprofen, ketorolac, loxoprofen, naproxen, tiaprofenic acid, suprofen, mefenamic acid, meclofenamic acid, phenylbutazone, azapropazone, metamizole, oxyphenbutazone, sulfinprazone, lornoxicam, meloxicam, piroxicam, tenoxicam, celecoxib, etoricoxib, lumiracoxib, parecoxib, rofecoxib, valdecoxib, lodine, nimesulide, and licofelone; prostaglandine receptor inhibitors; 5-lipoxygenase inhibitors, such as zileuton; 5-lipoxygenase activating protein inhibitors; leukotriene receptor antagonists, such as pobilukast, montelukast, pranlukast, roflumilast, and zafirlukast; bradykinin receptor antagonists; matrix metalloproteinase (MMP) inhibitors; anti-inflammatory monoclonal antibodies; and TNF receptor inhibitors; including any pharmaceutically acceptable salts, esters, isomers, stereoisomers, diastereomers, epimers, solvates or other hydrates, prodrugs, derivatives, or any other chemical or physical forms of active compounds comprising the respective active moieties.

The class or therapeutic category of anti-infective agents is herein understood as comprising compounds which are effective against bacterial, fungal, viral and protozoal infections, i.e. encompassing the classes of antimicrobials, antibiotics, antifungals, antivirals, antiprotozoals, and antiseptics.

Examples of useful antibiotics (including any pharmaceutically acceptable salts, esters, isomers, stereoisomers, diastereomers, epimers, solvates or other hydrates, prodrugs, derivatives, or any other chemical or physical forms of active compounds comprising the respective active moieties) are
- penicillins, all or not combined with beta-lactamase inhibitors (such as clavulanic acid, sulbactam, and tazobactam), including narrow-spectrum penicillins such as benzylpenicillin, phenoxymethylpenicillin, benzathine benzylpenicillin, procaine benzylpenicillin, clemizol benzylpenicillin, dibenzyletylenediamine benzylpenicillin; narrow-spectrum penicillinase-resistant penicillins, such as methicillin, oxacillin, cloxacillin, dicloxacillin, flucloxacillin, nafcillin, propicillin, mecillinam; narrow spectrum beta-lactamase-resistant penicillins, such as temocillin; and extended spectrum penicillins, such as ampicillin, amoxicillin, bacampicillin, pivampicillin, ticarcillin, azlocillin, piperacillin, apalcillin, carbenicillin, mezlocillin, and pivmecillinam;
- cephalosporins, including first generation cephalosporins, such as cefacetrile, cefadroxil, cefalexin, cephaloglycin, cefalonium, cefaloridine, cefalotin, cefapirin, cefatrizine, cefazaflur, cefazedone, cefazolin, cefradine, cefroxadine, ceftezole; second generation cephalosporins, such as cefonicid, cefprozil, cefuroxime, cefuroxime-axetil, cefuzonam, cefaclor, cefamandole, ceforanide, cefotiam, cefotiam-hexetil, loracarbef, cefbuperazone, cefmetazole, cefminox, cefotetan, cefoxitin; third generation cephalosporins, such as cefcapene, cefdaloxime, cefdinir, cefditoren, cefetamet, cefetamet-pivoxil, cefixime, cefmenoxime, cefodizime, cefoperazone, cefotaxime, cefpimizole, cefpodoxime, cefpodoxime-proxetil, cefteram, ceftibuten, ceftiofur, ceftiolene, ceftizoxime, ceftriaxone, ceftazidime, cefpiramide, cefsulodin, latamoxef; fourth generation cephalosporins, such as cefclidine, cefepime, cefluprenam, cefoselis, cefozopran, cefpirome, cefquinome, flomoxef; and further cephalosporins, such as cefaclomezine, cefaloram, cefaparole, cefcanel, cefedrolor, cefempidone, cefetrizole, cefivitril, cefmatilen, cefmepidium, cefovecan, cefoxazole, cefrotil, cefsumide, ceftioxide, cefuracetime, and ceftobiprole;
- carbapenems, including imipenem, imipenem-cilastatin, meropenem, doripenem, faropenem, tebipenem, ertapenem, panipenem, biapenem and ritipenem;
- monobactams, including aztreonam;
- aminoglycosides, such as amikacine, apramycin, arbekacine, capreomycin, gentamycin, hygromycin B, isepamycin, kanamycin, mupirocin, neomycin, netilmicin, paromomycin, spectinomycin, streptomycin, and tobramycin;
- macrolides, including erythromycin, azithromycin, clarithromycin, dirithromycin, dithromycin, roxithromycin, troleandomycin, carbomycin A, josamycin, kitasamycin, oleandomycin, spiramycin, tylosin, midecamycin, rapamycin, miocamycin, fluritromycin, rokitamycin, rosaramycin, and telithromycin;
- gyrase inhibitors or fluoroquinolones, including first generation fluoroquinolones, such as nalidixic acid, oxolinic acid, and piromidic acid; second generation fluoroquinolones, such as cinoxacin, flumequine, novobiocin, pipemidic acid, and rosaxacin; third generation fluoroquinolones, such as enoxacin, norfloxacin, nadifloxacin, ciprofloxacin, ofloxacin, fleroxacin, lomefloxacin, pefloxacin, temafloxacin, and uvofloxacin; and fourth generation fluoroquinolones, such as balofloxacin, caderofloxacin, clinafloxacin, difloxacin, garenaxacin, gatifloxacin, gemifloxacin mesylate, grepafloxacin, levofloxacin, moxifloxacin, olamufloxacin, pazufloxacin, rufloxacin, sitafloxacin, sparfloxacin, tosufloxacin, trovafloxacin, ecinofloxacin, and prulifloxacin;
- tetracyclins, including tetracycline, chlortetracycline, oxytetracycline, demeclocycline, doxycycline, clomocycline, lymecycline, meclocycline, methacycline, minocycline, penimepicycline, rolitetracycline, chelocardin, sancycline, apicycline, guamecycline, meglucycline, mepylcycline, pipacycline, etamocycline, penimocycline, and tigecycline;
- glycopeptides, including vancomycin, teicoplanin, ristocetin, avoparcin, oritavancin, ramoplanin, decaplanin, and peptide 4;
- polymycins, including polymyxin B, colistin, and surfactin;
- lincosamides, including lincomycin and clindamycin;
- streptogramins, including dalfopristin, quinupristin, pristinamycin, and virginiamycin;
- phenicols, including chloramphenicol, tiamphenicol, and florphenicol;
- rifamycins, including rifampicin, rifabutin, rifapentine, and rifaximin;
- nicotinic acid derivatives, including isoniazid, ethionamide, prothionamide, and pyrazinamide;
- nitroimidazoles, including metronidazole, timidazole, nimorazole and ornidazole;
- nitrofurans, including nifurfolin, nifuroxazide, nifuroxima, nifurzide, nitrofurantoin, and nitrofurazone;
- sulfonamides, including sulfacarbamide, sulfamazole, sulfamazone, sulfamethizole, sulfametopirazine, sulfametoxypiridazine, sulfametrol, succinylsulfathiazole, sulfisoxazole, sulfamethoxazole, sulfadiazine, phtalylsulfacetamide, phthalylsulfonazole, phtalylsulfathiazole, sulfasalazine, sulfoguanidine, sulfacetamide, silver sulfadiazine, mafenide acetate, sulfadoxine, sulfalen, cotrimoxazole, cotrimetrol, cotrimaxin, and cotetroxacin;
- further antibiotics, including plectasin, dalbavancin, daptomycin, ramoplanin, telavancin, bacitracin, tyrothricin, tygecycline, oxazolidinones (such as linezolide), fosfomycin, cycloserine, terizidon, inhibitors of dihydropteroate synthetase, sulfones, p-aminosalicylic acid, 2,4-diaminopirimidines (such as bromodiprim, pyrimethamine, tetroxyprim), trimethoprim, ranbezolid, ethambutole, dapsone, fucidinic acid, terizidone, ansamycin, lysostaphin, iclaprim, mirocin B17, clerocidin, filgrastim, and pentamidine;

Examples of useful antifungals are allylamines and thiocarbamates, including terbinafine, amorolfine, naftifine, butenafine, tolciclat, and tolnaftate; polyenes, including amphotericin B, natamycin, nystatin, flucocytosine, and rimocidin; azoles and triazoles, including bifonazole, clotrimazole, croconazole, econazole, fenticonazole, isoconazole, miconazole, axiconazole, sertaconazole, tioconazole, butoconazale, sulconazole, tioconazole, fluconazole, itraconazole, ketoconazole, voriconazole, ravuconazole, posaconazole, isavuconazole, and terconazole; echinocandins, including micafungin, caspofungin, and anidulafungin; further antifungals, including flucytosin, griseofluvin, ciclopirox olamine, haloprogin, and undecylenic acid.

Examples of useful antivirals are amantadine and derivatives, including tromantadine and rimantadine; neuraminidase inhibitors, including oseltamivir, zanamivir, and peramivir; nucleosides, including acyclovir, valaciclovir, penciclovir, famciclovir, brivudine, idoxuridine, trifluridine, vidarabine, ganciclovir, cidofovir, entecavir, and valganciclovir; antiretroviral agents, including zidovudine, abacavir, adefovir, didanosine, lamivudine, stavudine, zalcitabine, delavirdine, emtricitabine, efavirenz, loviride, nevirapine, indinavir, nelfinavir, ritonavir, saquinavir, amprenavir, lopinavir, atazanavir, fosamprenavir, tipranavir, darunavir, adefovir, enfuvirtide, loviride, and tenofovir; further antiviral agents, including foscarnet, ribavirin, arbidol, docosanol, edoxudine, fomivirsen, fosfonet, ibacitabine, imunovir, imiquimod, inosine, interferons, lysozyme, maraviroc, moroxydine, nexavir, pleconaril, podophyllotoxin, vicriviroc, and viramidine; fixed combinations of antivirals, including atripla, combivir, emtricitabine, trizivir, and truvada.

Examples of useful antiprotozoal drugs include for example pentamindine, cotrimoxazole, metronidazole, tinidazol, nimorazol, and ornidazol.

Examples of useful antiseptics are acridine derivatives, iodine-povidone, benzoates, rivanol, chlorhexidine, quaternary ammonium compounds, cetrimides, biphenylol, clorofene, and octenidine.

Examples of useful prostaglandins are prostacyclin, epoprostenol, treprostinil, and iloprost.

Examples of useful endothelin receptor agonists are bosentran, sitaxsentan, ambrisentan, and darusentan.

Examples of useful phosphodiesterase inhibitors are non-selective methylxantines, such as theophylline and pentoxyphylline; and selective PDE isoenzyme inhibitors, such as amrinone, cilostazol, benzafentrine, milrinone, enoximone, motapizone, zardaverine, tolafentrine, rolipram, cilomast, roflumilast, sildenafil, vardenafil, and tadalafil.

Examples of useful beta-2-sympathicomimetics are short-acting β₂ agonists, such as salbutamol (albuterol), levalbuterol, terbutaline, pirbuterol, procaterol, metaproterenol, fenoterol, bitolterol, and clenbuterol; and long-acting β₂ agonists, such as salmeterol, formoterol, bambuterol, carmoterol, arformoterol, indacaterol, and picumeterol. Examples of useful decongestants and vasoconstrictors are alfa-1-sympathicomimetics, such as indanazoline, naphazoline, oxymetazoline, tetryzoline, tramazoline, xylometazoline, phenylephrine, phenoxazoline, epinephrine, ephedrine, isoprenaline, and hexoprenaline.

Examples of useful anticholinergics are short-acting anticholinergics, such as ipratropium, oxitropium, and trospium; and long-acting anticholinergics, such as tiotropium, revatropate, glycopyrronium, and aclidinium.

Examples of useful immunomodulators are the above named glucocorticoids and non-steroidal glucocorticoid receptor activators; immunosuppressive monoclonal antibodies, such as omalizumab, infliximab, adalimumab, and etanercept; cyclosporine, tacrolimus, sirolimus (rapamycin), mycophenolat, dimethylfumarate, ethylhydrogenfumarat, methotrexate, azathioprin, interferones (alpha, beta, gamma), tumor necrosis factors, cytokines, interleukins, echinacea extract, and pelargonium extract.

Examples of useful mucolytics are acetylcysteine, ambroxol, bromhexine, carbocysteine, gluthation, nacystelyn, dornase alpha, mugwort, bromelain, papain, clerodendrum, guaifenesin, cineol, guajakol, myrthol, mesna, P2Y2-agonists (such as denufosol), heparinoids, sodium chloride, drugs that influence the uptake of chloride and sodium, such as for example N-(3,5-diamino-6-chlorpyrazin-2-carbony)-N'-{4-[4-(2,3-dihydroxypropoxy)-phenyl]butyl}guanidin-Methanesulfonat (PARION 552-02), tyloxapol, lecithin, and recombinant surfactant proteins.

Examples of useful antihistaminica are diphenhydramine, carbinoxamine, doxylamine, clemastine, dimenhydrinate, pheniramine, chlorphenamine, dexchlorphenamine, brompheniramine, triprolidine, cyclizine, chlorcyclizine, hydroxyzine, meclizine, promethazine, alimemazine, cyproheptadine, azatadine, ketotifen, azelastine, levocabastine, olopatadine, epinastine, emedastine, acrivastine, astemizole, cetirizine, loratadine, mizolastine, terfenadine, fexofenadine, levocetirizine, and desloratadine.

Examples of useful mast-cell stabilising agents are cromoglycate, nedocromil, and lodoxamide.

Examples of potentially useful antiallergic agents include the afore-mentioned glucocorticoids, mast-cell stabilizing agents, anti-histaminica, leukotriene receptor antagonists, ziluton, omalizumab, and heparinoids.

Examples of useful tumor growth inhibitory agents are alkylants, such as nimustine, melphanlane, carmustine, lomustine, cyclophosphosphamide, ifosfamide, trofosfamide, chlorambucil, busulfane, treosulfane, prednimustine, thiotepa, dacarbazine, and complexes of transition group elements (e.g. Ti, Zr, V, Nb, Ta, Mo, W, Pt) such as carboplatinum, oxiplatinum, cis-platinum, metallocene compounds such as titanocendichloride; antimetabolites, such as cytarabine, fluorouracil, methotrexate, mercaptopurine, tioguanine, hydroxycarbamide, pemetrexed, and gemcitabine; alkaloids, such as vinblastine, vincristine, vindesine, and vinorelbine; antitumoral antibiotics, such as alcarubicine, bleomycine, dactinamycine, daunorubicine, doxorubicine, epirubicine, idarubicine, mitoxantron, mitomycine, and plicamycine; and further tumor growth inhibitory agents, such as erlotinib, gefitinib, methotrexate, paclitaxel, docetaxel, amsacrine, estramustine, etoposide, beraprost, procarbazine, temiposide, vandetanib, poly-ADP-ribose-polymerase (PRAP) enzyme inhibitors, banoxantrone, premetrexed, bevacizumab, and ranibizumab.

Examples of useful wound-healing agents are dexpantenol, allantoin, vitamins, hyaluronic acid, alpha-antitrypsin, anorganic and organic zinc salts/compounds, and salts of bismuth and selen.

Examples of useful local anaesthetics are benzocaine, tetracaine, procaine, lidocaine and bupivacaine.

Examples of useful antioxidants are superoxide dismutase, acetylcysteine, vitamin C, vitamin E (tocopherols), catalase, reduced glutathione, peroxidases, uric acid, β-carotene, NOX inhibitors, xanthin oxidase inhibitors, pyruvate and gluconate salts.

Examples of useful plant extracts and ingredients are extracts from for example chamomile, hamamelis, Echinacea, calendula, thymian, papain, pelargonium, and pine trees; and essential oils, such as myrtol, pinen, limonen, cineole, thymol, menthol, camphor, tannin, alpha-hederin, bisabolol, lycopodin, resveratrol, vitapherole and anti-oxidative ingredients of green thee.

Examples of useful angiotensin converting enzyme (ACE) inhibitors include captopril, lisinopril, perindopril, trandolapril, and cilazapril.

Useful potassium channel openers are for example cromakalim, levocromakalim, and pinacidil.

Examples of potentially useful tachykinin and kinin antagonists are nolpitantium, saredutant, nepadutant, and osanetant.

Antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides, depending on their composition, useful for the treatment of many diseases. Various compounds are currently clinically evaluated, such as ALN-RSV01 to treat the respiratory syncytical virus, AVE-7279 to treat asthma and allergies, TPI-ASM8 to treat allergic asthma, and 1018-ISS to treat cancer.

Examples of potentially useful peptides and proteins include amino acids such as L-arginine and L-lysine, antibodies against toxins produced by microorganisms, and antimicrobial peptides such as cecropins, defensins, thionins, and cathelicidins.

For any of these and other explicitly mentioned examples of drug substances which are potentially useful for carrying out the invention, the compound names given herein should be understood as also referring to any pharmaceutically acceptable salts, esters, isomers, stereoisomers, diastereomers, epimers, solvates or other hydrates, prodrugs, derivatives, or any other chemical or physical forms of the respective compounds comprising the respective active moieties.

The active compound comprised in the aerosol used for the method of the invention may be a drug substance which is useful for the prevention, management, or treatment of any disease, symptom, or condition affecting the nose, the sinuses and/or the osteomeatal complex, such as acute and chronic sinusitis, such as allergic sinusitis, seasonal sinusitis, bacterial sinusitis, fungal sinusitis, viral sinusitis, frontal sinusitis, maxillary sinusitis, sphenoid sinusitis, ethmoid sinusitis, vacuum sinusitis; acute and chronic rhinitis, such as allergic rhinitis, seasonal rhinitis, bacterial rhinitis, fungal rhinitis, viral rhinitis, atrophic rhinitis, vasomotor rhinitis; any combination of rhinitis and sinusitis (i.e. rhinosinusitis); nasal polyps, nasal furuncles, epistaxis, wounds of the nasal or sinunasal mucosa, such as after injury or surgery; and dry nose syndrome; nasal or sinunasal conditions related to lower respiratory tract diseases such as asthma and cystic fibrosis (CF); and nasal or sinunasal conditions related to ear diseases such as inflammation of the middle ear (otitis media), inner ear, external ear, ear canal and Eustachian tube. Furthermore, the active compound may be a drug used for the treatment of upper and lower respiratory tract diseases such as inflammation, allergy, oropharyngeal infections, laryngotracheobronchitis, bronchitis, bronchiolitis, such as diffuse bronchiolitis and bronchiolitis obliterans, bronchiectasis, alveolitis, pneumonia, such as community acquired pneumonia (CAP), hospital acquired pneumonia (HAP), and ventilator associated pneumonia (VAP), pulmonary infection with or without acute exacerbations, such as bacterial, viral, fungal, and protozoal infections of the respiratory tract, and respiratory infections in HIV patients, asthma, chronic obstructive pulmonary disease (COPD), emphysema, pneumocystis, sarcoidosis, tuberculosis, nontuberculous mycobacterial pulmonary diseases, pulmonary ciliary dyskinesia, parenchymatic and/or fibrotic diseases or disorders including cystic fibrosis, interstitial lung diseases, pulmonary hypertension, whooping cough, respiratory distress syndrome, interstitial lung disease, meconium aspiration syndrome, lung obstructions, lung cancer, lung transplantation, and graft rejection after lung, stem or bone marrow transplantation. The active compound comprised in the aerosol may also be useful for prevention, management, or treatment of any systemic or brain disease, symptom, or condition.

The aerosolisable fluid composition may further comprise excipients, such as one or more solvents, co-solvents, acids, bases, buffering agents, osmotic agents, stabilizers, antioxidants, taste-masking agents, clathrate- or complex-forming compounds, polymers, flavours, sweetening agents, ionic and non-ionic surfactants, thickeners, colouring agents, fillers, and bulking agents.

Solvents and co-solvents, other than water, should be avoided if possible if the composition is intended for inhalation. If the incorporation of a solvent cannot be avoided, the excipient should be selected carefully and in consideration of its physiological acceptability. For example, if the composition is designated for the treatment of a life-threatening disease, the use of some limited amount of ethanol, glycerol, propylene glycol or polyethylene glycol as a non-aqueous solvent may be acceptable. According to the currently more preferred embodiments, however, the composition is substantially free of these solvents, and in particular of glycerol, propylene glycol or polyethylene glycol.

In the embodiments shown in the figures, the one end of the fluid container 18 can be securely and tightly closed with a screw cap (not shown). At its other end, opposite the screw cap, the fluid container may have a tapered portion 22 that tapers towards a fluid chamber 24, as can be seen in Fig. 4. The fluid chamber 24 may be sealed by a sealing lip (not shown) that forms a part of the chamber 24 and is tightly pressed against a membrane 30. The membrane 30 is provided with a plurality of minute openings or holes with diameters in the micrometer range that fully penetrate the membrane 30. Furthermore, the membrane 30 can be vibrated (or oscillated), for example with the use of a piezoelectric element (not shown), such that the direction of the vibrations is perpendicular to the plane of the membrane 30. A terminal element for enabling supply of electrical power and control of the membrane 30 may be integrally formed with the body of the aerosol delivery device 10. By inducing such vibrations in the membrane 30, fluid contained in the fluid chamber 24 is passed through the minute openings of the membrane 30 and nebulised into the nebuliser chamber 32 formed at the other side (opposite the fluid chamber 24) of the membrane 30. In this way, the fluid chamber 24 and the membrane 30 together form a vibrating membrane nebuliser device (aerosol generator) 3. A detailed description of this common concept is given, for example, in US 5 518 179. A control (not shown) comprises a computer and a first control element (not shown), such as a transistor, that is connected to the membrane 30 for stopping the membrane vibration and hence the aerosol generation before an optional step of pulsating (vibrating) the aerosol may be carried out.

A circulation portion 36 is formed between the membrane 30 and the body (not shown) of the aerosol delivery device 10 that allows for the passage of a gas, i.e., air in the present embodiments, supplied from the compressor 1 (not shown in Fig. 4) through the connector 12. In the embodiments shown in Figs. 1 to 4, the gas compressor 1 is used as the gas conveying element (or transport flow producer) and a sinus wave generator (not shown) that is also connected to the connector 12 is optionally used as the pulsator 2, as will be further explained in the following. The control (not shown) further comprises a second control element (not shown), that is disposed on the compressor 1 for activating and deactivating the compressor 1 after predetermined periods of time. In further embodiments, the second control element may be magnetical, electrical and/or mechanical, such as a valve, regulator and/or controller. The second control element can be controlled, for example, with the computer of the control.

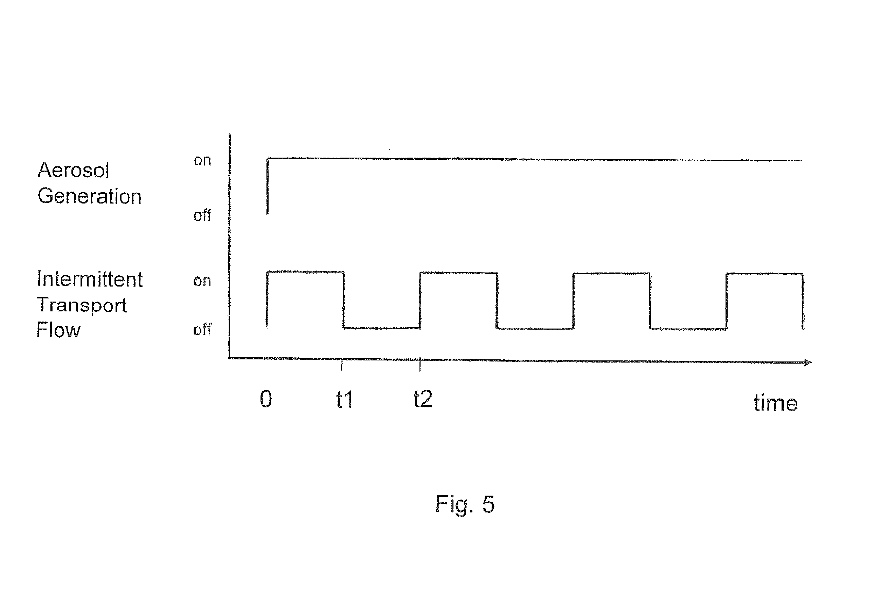

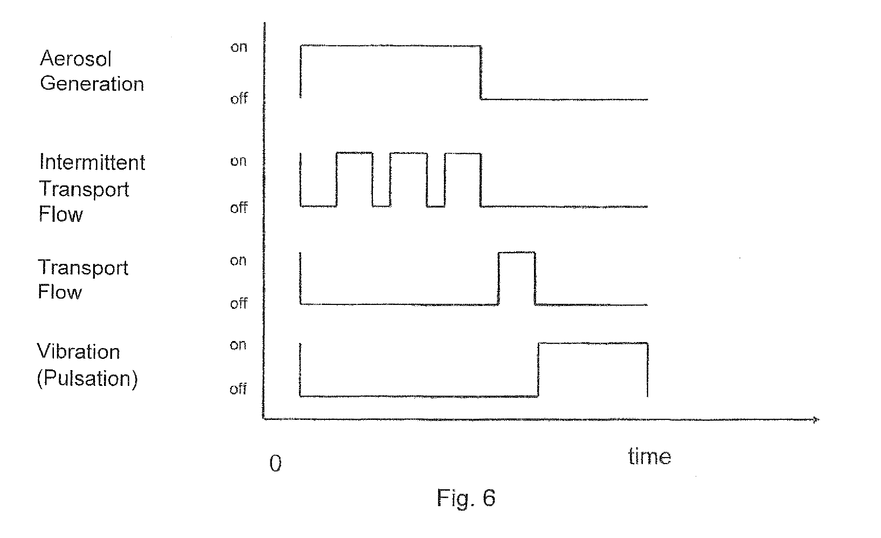

Next, different examples of the operation of the above described aerosol delivery device 10 of the embodiments shown in Figs. 1 to 4 will be explained. Figures 5 and 6 show flow diagrams illustrating the sequence of the different steps carried out for depositing a certain amount of an aerosol at a target area, such as the paranasal sinuses. First, the fluid container 18 is filled, for example, with 15 ml of an aerosolisable fluid that comprises an active compound, such as an anti-allergic drug, and tightly sealed with the screw cap (not shown). Then, the nosepiece 16 of the adaptation element 14 is inserted into a nostril of a patient 100 who has a medical condition to be treated. Since no counterpressure element, such as a nose plug, placed in the patient's other nostril is required for the operation of the aerosol delivery device of the present embodiments, the patient can inhale and exhale freely through the other nostril while the treatment is being carried out.

Subsequently, in the example of Fig. 5, which shows operation of the aerosol delivery device 10 in a pure intermittent mode, the aerosol generation is started by oscillating the membrane 30 so that it nebulises a certain amount of the fluid received in the container 18 into the nebuliser chamber 32 and, at the same time, a constant transport flow of gas (air) is supplied at a flow rate of 0.5 to 3 L/min by the gas compressor 1. The flow rate was measured with the Flow Sensor FBAL001DU from Sensortechnics, which has sufficiently short response times of 1-3 ms. As can be seen in Fig. 4, the plane of the membrane 30 is substantially perpendicular to the direction of aerosol transport (direction of arrow A in Fig. 4) towards the adaptation element 14, so that the risk of any aerosol loss at the walls of the aerosol delivery device 10 due to impaction is minimised. The air supplied from the compressor circulates around the membrane 30 through the circulation portion 36 and mixes with the nebulised fluid in the nebuliser chamber 32, thus generating an aerosol.

After a predetermined first period of time (t1) of 200 ms, the constant transport flow is stopped and then induced again after a predetermined second period of time (t2) of 200 ms by operation of the second control element (not shown) which is controlled, for example by the computer of the control. This cycle of stopping and inducing the transport flow is periodically repeated three times before the aerosol deposition in the desired location, e.g., the paranasal sinuses, is finished. Subsequently, the therapeutic treatment can be repeated until it is completed and the aerosol delivery device can be removed from the patient's 100 nostril.

Figure 6 shows operation of the aerosol delivery device 10 in a combined mode, which combines an intermittent mode, such as that shown in Fig. 5, with a subsequent pulsation mode. After the operation in the intermittent mode, once a certain desired amount of an aerosol, such as 0.1 to 3.0 times the volume of the desired location (e.g., the nasal cavity), for example 8 ml, has been generated inside the aerosol delivery device 10, the first control element (not shown) is operated, for example by the computer of the control, in order to halt the vibration of the membrane 30 and hence stop the aerosol generation. Specifically, this step may be, for example, carried out by monitoring the amount of fluid remaining in the fluid container 18 with a sensor element (not shown) placed within the container 18 and interrupting the supply of electrical power to the membrane 30, when the remaining amount of fluid has reached a predetermined value. Depending on the type of aerosol, the therapeutic treatment to be performed etc., the duration of the aerosol generating step may vary, typically between 400 and 1200 ms.

Subsequently, in the operation example of Fig. 6, an optional additional aerosol transporting step is performed after the aerosol generation has been stopped, in order to empty the device 10 of any remaining aerosol. However, this additional step may also be omitted. After a predetermined period of time, this aerosol transporting step is stopped by switching off the gas compressor 1. This period of time may be set as the time required for the aerosol to arrive at the desired location, e.g., the paranasal sinuses, which may, for example, be identified by monitoring the aerosol flow rate and the time from the start of the additional transporting step, taking into account the volume of the aerosol delivery device 10. In the present operation example, the volume of the generated and transported aerosol is 8 ml, which is about half the average volume of the nasal cavity (15 ml) of an adult patient. Hence, the nasal cavity is only half filled with aerosol, reducing the amount of inhaled aerosol that does not reach the paranasal sinuses and thus does not contribute to the therapeutic treatment.

After the additional aerosol transporting step has been stopped, as described above, a pulsation of the transported aerosol is triggered. As mentioned above, the pulsator 2 of the present embodiments is a sinus wave generator that is connected to the connector 12 and capable of generating flow oscillations with frequencies in the range of 1 to 200 Hz. In the present example, the transported aerosol is subjected to a pulsation with a frequency of 25 Hz and an amplitude (pulsation flow rate) of 10 L/min for a period of 0.5 s. After this pulsation step has been carried out, the therapeutic treatment can be repeated until it is completed and the aerosol delivery device can be removed from the patient's 100 nostril.

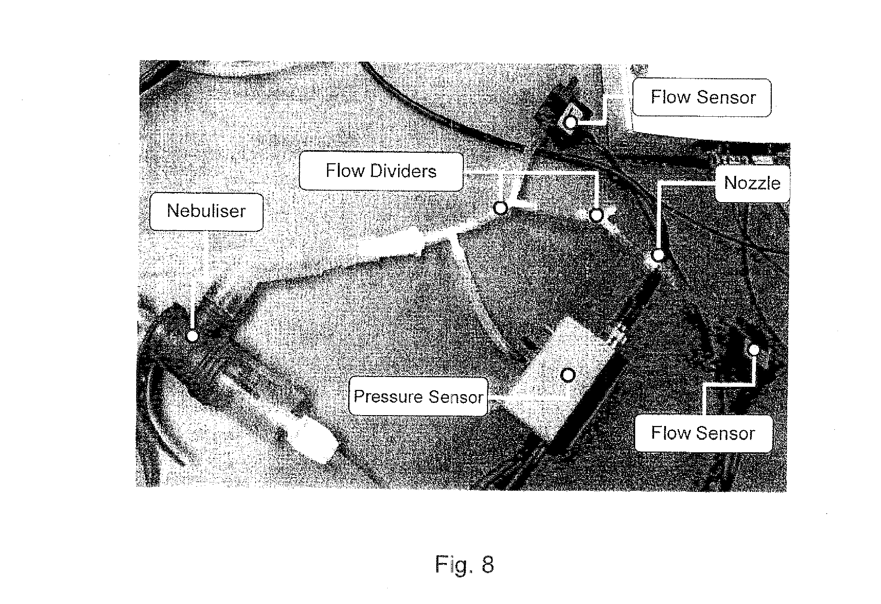

The amplitude (pulsation flow rate) was measured by using two Flow Sensors FBAL001DB from Sensortechnics, which exhibit sufficiently short response times of 1-3 ms and are capable of measuring both positive and negative flows. Since these flow sensors are only specified for flow rates of 0-1 L/min, the measurement range had to be appropriately expanded. This aim was achieved by employing conventional flow dividers and a LüerLock tube connector as a nozzle. In this way, the high pulsation flow rates of the aerosol delivery device (nebuliser) 10 could be sufficiently attenuated so as to enable reliable operation of the flow sensors. A perspective view of the measurement set-up used is shown in Fig. 8. In order to determine the relation between the attenuated measured flow rates and the actual flow rates at the nebuliser outlet, thus allowing for a reliable and precise measurement of these actual flow rates, the set-up was calibrated prior to the measurement. For this purpose, various constant flow rates in the range of 0-20 L/min were adjusted using a needle valve and the corresponding flow sensor signals were measured. For negative flows, the flow sensors were reversely arranged (i.e., turned around so as to reverse the flow measurement direction) and the calibration was performed in the same manner as for positive flows.

By pulsating the transported aerosol when it has reached a desired location, the impaction of aerosols on the walls of the aerosol delivery device and/or the nasal cavity can be significantly reduced, as has been explained in detail above.

Experimental studies on the efficiency of aerosol deposition in the paranasal sinuses were performed, using an aerosol delivery device 10 as schematically shown in Figs. 1 and 4 and a human nasal cast model with a fixed sinus volume of 12 ml and varying ostium diameters (0.5 to 10 mm).

The nasal cast model is based on the anatomical shapes and dimensions of the human nasal cavity and the nasal passage and was built from plastic (polyoxymethylene). In this model, the paranasal sinuses are simulated by 6 exchangeable glass bottles, 3 on either side, representing the frontal, maxillary, and sphenoid sinuses, respectively. Exchangeable, artificial ostia of 10 mm length were used to connect the artificial sinus cavities to the nose model. Moreover, the model has two openings representing artificial nostrils and one opening for the simulation of the pharynx which connects the nasal cavity with the trachea. The model also contains silicone made inlays in the nasal cavities in order to mimic the narrow cross sectional areas of the nasal turbinates. These inlays have, like the human nose, a high filter efficiency and allow the comparison of various devices under more realistic conditions.

The aerosol delivery device 10 was operated in a pure intermittent mode, such as that shown in Fig. 5, with a transport flow rate of 2.0 L/min, a first period of (on) time of 200 ms and a second period of (off) time of 50 ms. Four cycles of inducing and stopping the transport flow were periodically performed.

In a further trial, the aerosol delivery device 10 was operated in a pure combined mode, such as that shown in Fig. 6, with a transport flow rate of 2.0 L/min, a first period of (on) time of 200 ms and a second period of (off) time of 50 ms. Four cycles of inducing and stopping the transport flow were periodically performed and finally a further transport of 2.0 L/min and a pulsation period with a frequency of 25 Hz and a flow amplitude of 10 L/min followed.

For the aerosol generation, an aqueous liquid solution of levofloxacin comprising 10 wt.-% of the active ingredient was prepared. The inactive ingredients were xylitol (2 wt.-%), magnesium gluconate (10.5 wt.-%), and water.

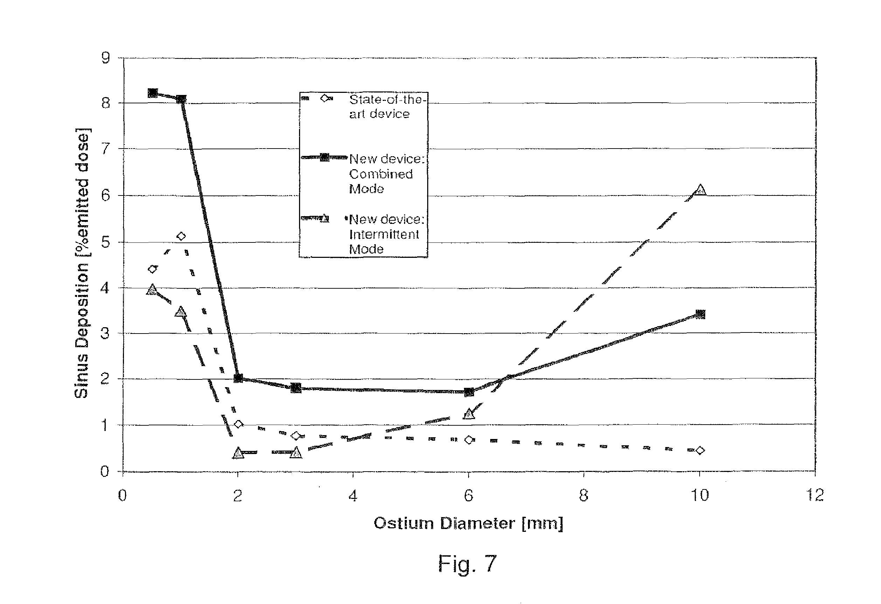

Characteristic results of these studies are shown in Fig. 7. As can be seen from this figure, device operation in the intermittent mode allows for highly efficient aerosol deposition in the paranasal sinuses, in particular for large ostia diameter and device operation in the combined mode allows efficient aerosol deposition in the paranasal sinuses over all ostia diameter. Both working modes (intermittent mode and combined mode) work better than the known state-of-the-art device with continued pulsation (vibration) and continued transportation of the aerosol. Hence, these operating methods are particularly advantageous for treating all individual patients with normal or enlarged ostia, for example, after functional endoscopic sinus surgery (post FESS), as has been discussed above.

In a further clinical trial the aerosol delivery device 10 was operated in a combined mode and an intermittent mode. The combined mode was such as that shown in Fig. 6, with a transport flow rate of 3.0 L/min, a first period of time of 200 ms and a second period of time of 50 ms. Four cycles of inducing and stopping the transport flow were periodically performed and finally a further transport of 3.0 L/min and a pulsation period with a frequency of 25 Hz and a flow amplitude of 10 L/min followed. To this combined mode was compared the intermittent mode, which was such as that shown in Fig. 6, with a transport flow rate of 2.0 L/min, a first period of time of 200 ms and a second period of time of 50 ms. Four cycles of inducing and stopping the transport flow were periodically performed.

The method of the clinical trial was: Sinus air ventilation, nasal and paranasal aerosol deposition as well as nasal clearance of aerosols delivered by different pulsating airflows, such as a combined mode in comparison to intermittent mode. This was studied in 12 healthy volunteers using gamma camera imaging with 81mKr gas and 99mTc-DTPA aerosol. Masking techniques were used to visualize and quantify nasal and sinus aerosol deposition and retention.

The following results have been found: Ventilation of the paranasal sinuses by pulsating airflows was confirmed in all volunteers. Aerosol deposition could be observed in the maxillary and sphenoid sinuses with a total sinus deposition up to 24% of the deposited activity. Nasal masking with a lead shield enabled the clear detection of activity in the maxillary sinuses.

Exemplary results for two different volunteers with and without "FESS" are shown in the following table. Both volunteers inhaled with a vibrating membrane nebuliser as aerosol delivery device and used two different pulsation modes: combined mode and intermittent mode. The operating method with the combined mode is particularly advantageous for treating all individual patients with normal or enlarged ostia. The intermittent mode is working especially well in patients with larger ostia, such as post FESS.

| working mode | proband 1 | proband 2 |
|---|---|---|
| | post FESS volunteer | normal volunteer |
| intermittent mode | 24% | 0% |
| combined mode | 14% | 6% |

Table 1 shows the total sinus deposition in % of the deposited activity in different modes and probands.

## Claims

1. A method for operating an aerosol delivery device (10), comprising the steps of:
- generating a predetermined amount of an aerosol in the device (10), and
- inducing a transport flow in the device (10) for transporting at least a portion of the predetermined amount of the aerosol outside the device (10),
wherein, during the aerosol generating step, the transport flow is first induced, then stopped after a predetermined first period of time and then induced again after a predetermined second period of time.

2. The method according to claim 1, wherein the total volume of transported gas/aerosol bolus during one period step is less than 50 ml, preferably between 5 ml and 20 ml.

3. The method according to claim 1 or 2, further comprising a step of pulsating the aerosol after the aerosol generating step.

4. The method according to claim 3, wherein the transport flow is stopped during the step of pulsating the aerosol.

5. The method according to claim 3 or 4, wherein the duration of the step of pulsating the aerosol is in the range of 0.1 - 15.0 s, preferably in the range of 0.5 - 1.0 s.

6. The method according to any one of claims 3 to 5, wherein the pulsation of the aerosol has a frequency in the range of 1 - 200 Hz.

7. The method according to any one of the preceding claims, wherein the aerosol is a pharmaceutical aerosol for the delivery of an active compound.

8. The method according to any one of the preceding claims, wherein the aerosol delivery device (10) is a vibrating membrane nebuliser.

9. The method according to any one of the preceding claims, further comprising a step of low-frequency pulsation with a frequency in the range of less than 60 Hz, preferably between 5 to 40 Hz, most preferred between 10 and 20 Hz.

10. An aerosol delivery device (10) comprising:
- an aerosol generator (3) for generating an aerosol in the device (10),
- a gas conveying element (1) for inducing a transport flow in the device (10) for transporting at least a portion of the generated aerosol outside the device (10), and
- a control configured, during the aerosol generating step, to first activate the gas conveying element (1), then deactivate the gas conveying element (1) after a predetermined first period of time and then activate the gas conveying element (1) again after a predetermined second period of time, so that the transport flow is first induced, then stopped after the first period of time and then induced again after the second period of time.

11. The aerosol delivery device (10) according to claim 10, further comprising a pulsator (2) for pulsating the aerosol, wherein the control is configured to activate the pulsator (2) after deactivating the aerosol generator (3).

12. The aerosol delivery device (10) according to claim 11, wherein the control is configured to deactivate the gas conveying element (1) during the pulsation of the aerosol.

13. The aerosol delivery device (10) according to claim 11 or 12, further comprising a switch unit for switching the control between an operating mode which includes activating the pulsator (2) after deactivating the aerosol generator and an operating mode in which the pulsator (2) is not activated.

14. The aerosol delivery device (10) according to any one of claims 10 to 13, wherein the aerosol generator (3) is a vibrating membrane nebuliser.

15. The subject-matter according to any one of the preceding claims, wherein, preferably at least three times, the transport flow is periodically induced and stopped during the aerosol generating step.

16. The subject-matter according to claims 3 to 8 and 11 to 15, wherein the pulsation of the aerosol has a used maximal pulsation flow greater than 8 L/min, preferably greater than 12 L/min and more preferably greater than 14 L/min.

17. The subject-matter according to claim 15 or 16, wherein the frequency of periodically inducing and stopping the transport flow is less than 10 Hz, preferably between 1 and 10 Hz.

18. The subject-matter according to any one of the preceding claims, wherein the first period of time and/or the second period of time is in the range of 50 - 300 ms.

19. The use of the aerosol delivery device (10) according to any one of claims 10 to 18 for the method according to any one of claims 1 to 9 or 15 to 18 as dependent on claims 1 to 9.
